# EUROPEAN PATENT APPLICATION

(11) **EP 2 327 688 A1**
(43) Date of publication of application: **01.06.2011**
(21) Application number: 09812735.0
(22) Date of filing: 10.09.2009
(51) Int. Cl.: C07C 245/02, B01J 21/00, B01J 23/00, B01J 23/52

(54) **PREPARATION OF AZO COMPOUNDS WITH SOLID CATALYSTS**

(30) Priority: 15.09.2008 ES 200802659
(71) Applicant: Universidad Politécnica De Valencia (UPV) (50%), 46022 Valencia (ES); Consejo Superior de Investigaciones Cientificas (CSIC), 28006 Madrid (ES)
(72) Inventor: CORMA CANÓS, Avelino, E-46022 Valencia (ES); GARCÍA GÓMEZ, Hermenegildo, E-46022 Valencia (ES); GRIRRANE, Abdessamad, E-46022 Valencia (ES)
(74) Representative: Pons Ariño, Angel
(86) International application number: PCT/ES2009/070378
(87) International publication number: WO 2010/029208

(57) **Abstract**

The present invention refers to a procedure for preparing azo compounds comprising a reaction between at least: one amine or polyamine, molecular oxygen, a catalyst comprising at least one support selected from at least a metal oxide of one of the elements of the groups 3, 4, 5, 6, 8, 9, 11 and 13, silica, an anionic laminar compound of hydrotalcite type or its derivatives, active carbon or an organic polymer. In addition, said catalyst may contain nanoparticles of gold.

## Description

### Field of the invention

The present invention relates to heterogeneous solid catalysts based on nanoparticulated metal oxides containing or not containing gold and in different supports containing or not containing gold nanoparticles capable of stimulating the formation of azo compounds and, in particular, symmetric or asymmetric aromatic azo compounds by means of a reaction of pure amines, mixtures of amines or polyamines, with atmospheric oxygen as oxidizing agent. These catalysts may operate in continuous processes or in processes per load in which case it is possible to recover the solid by filtration and reuse it in a new reaction without any loss of its catalytic activity. When these catalysts contain gold in the form of nanoparticles, it is possible to couple the oxidation process of the amines with a previous process consisting of the formation of these amines by means of hydrogenation of nitro compounds. In this way, a single catalyst may effect the transformation of the nitro compounds into azo compounds.

### State of the art

Azo compounds have in common the presence of a -N=N- double bond (Figure 1) and may be aliphatic or aromatic depending on whether the third valences of the nitrogens are saturated with alkyl or aryl groups. In addition, the azo compounds may be classified into symmetric and asymmetric ones depending on whether the substituents of each one of the nitrogens are identical or different.

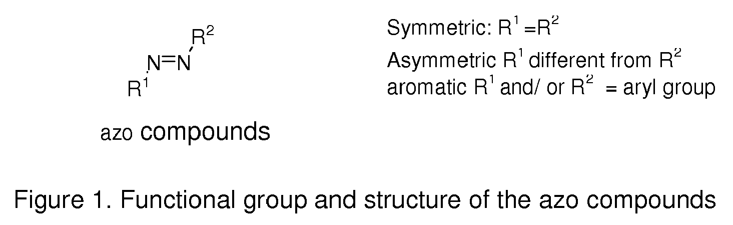

Azo compounds are used as generators of carbon free radicals, such as pigments, dyes, food additives, as pharmaceutical compounds and as ligands of metals. The azo compounds represent an intermediate state of oxidation between the nitro compounds and the amines and, therefore, may be obtained from compounds of any of these families (Figure 2). The reactions are completed by using stechiometric amounts of oxidizing or reducing agents often containing transition or reactive metals which are detrimental to the environment. Until now, no state of the art process has been disclosed which uses heterogenic catalysis, takes place with a high conversion level and has high selectivity.

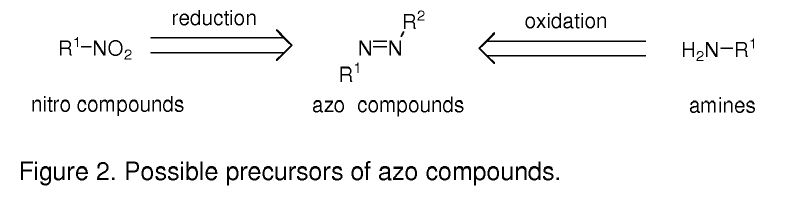

The azo compounds may be prepared from amines by means of a reaction with oxidizing agents such as, for example, lead tetraacetate. Azo compounds may also be obtained by reduction of nitro compounds with metals such as lead, for example. Aromatic asymmetric azo compounds may be prepared by means of a coupling reaction of diazonium salt with an activated aromatic compound versus an electrophilic substitution reaction. In this case, the usual form of preparation of the diazonium salt is by a reaction with nitrites in acid medium. In this case, stechiometric amounts of nitrite acting as a moderate oxidant are also required.

In the process described according to the present invention, heterogeneous and reusable catalysts are used for completing the formation of azo compounds by aerobic oxidation starting from amines. In addition, it has been observed that, in the case when the catalysts comprise gold nanoparticles, they noticeably increase the activity and the selectivity of said process. Therefore, the process of the invention makes it possible to obtain azo aliphatic and aromatic compounds of amines and polyamines with a high yield and high purity, while the heterogeneous nature of the solid catalyst facilitates its separation, recovery and reuse.

### Detailed description of the invention

The present invention relates to a preparation process of azo compounds which comprises the reaction between at least:
- one amine or polyamine,
- molecular oxygen,
- a catalyst formed by at least one support selected from among at least one metal oxide of one of the elements of groups 2, 3, 4, 5, 6, 8, 9, 11 and 13, silica, anionic laminar compound of hydrotalcite type or its derivatives, active carbon and an organic polymer.

Azo compounds are currently obtained by means of non-catalytic reactions which use stechiometric amounts of metals or metal compounds or other reagents. The formation processes of azo compounds that have been disclosed until now do not comply with the principles of *"green chemistry"* which require the development of chemical processes that do not produce by-products with a negative effect on the environment. The present invention discloses a process in which heterogeneous and reusable catalysts are used for completing the formation of azo compounds by aerobic oxidation starting from amines. The reagent consumed in this process is oxygen which gives water as a by-product. In addition, alternatively, in the case of those catalysts that may contain gold, the starting substrate of the synthesis could be a nitro compound, which is subjected first to a selective catalytic hydrogenation of the nitro group, and then, in the same or a different reactor and with the same or a different gold catalyst, could undergo an oxidation subsequent to the azo compound. Figure 3 summarizes the sequence of the synthesis.

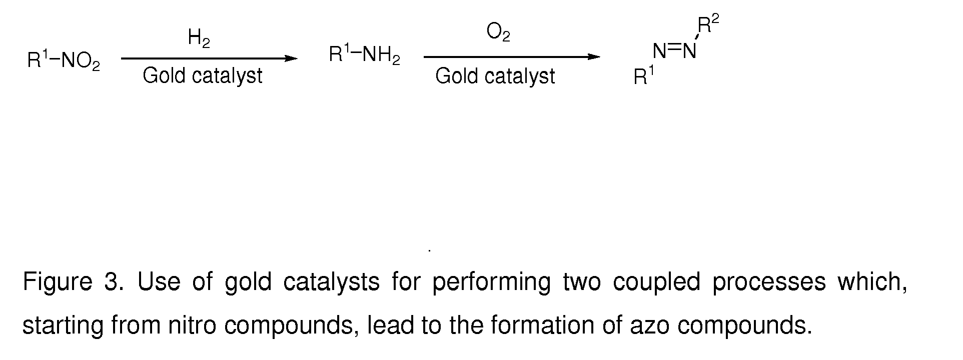

According to a specific embodiment of the present invention, the catalyst support used is a metal oxide of one of the elements of groups 3, 4, 5, 6, 8, 9, 11 and 13. Preferably, said metal oxide is selected from among TiO₂, CeₓO_{y}, Al₂O₃, MgO, CaO, CuₓO_{y}, COₓO_{y}, FeₓO_{y}, CrₓO_{y}, Y₂O₃, ZrO₂ and combinations thereof.

According to a preferred embodiment, the metal oxide is titanium oxide. According to another preferred embodiment, the metal oxide is cerium oxide. According to another preferred embodiment, the metal oxide is yttrium oxide. According to another preferred embodiment, the metal oxide is iron oxide. According to another preferred embodiment, the metal oxide is zirconium oxide.

In addition, according to the process of the present invention, the particle size of the oxide should be, preferably, between 1 nm and 50 nm.

According to a particular embodiment, the above-mentioned metal oxides may further contain a small percentage of other (metal or non-metal) element(s) which may act as doping agents. These elements are preferably selected among elements of groups 9, 10, 11 and 12. Some non-limiting examples of these elements may be Pt, Pd, Fe, Cu, La, Pr, Au, N, C, S and combinations thereof.

According to a particular embodiment of the present invention, the nanoparticulated titanium oxide (average particle size of 25 nm), predominantly in anatase phase (80%), catalyzes the formation of aniline in azobenzene with conversion of 97% and selectivity of 99%. In another particular embodiment, the nanoparticulated cerium oxide (average particle size of 5 nm) is capable of producing a conversion of 90% and selectivity with respect to the azobenzene of 59%.

In the process of the present invention, the catalyst support may be at least a microporous material, preferably a zeolytic material. Also, in particular, the support may be at least a structured mesoporous material.

According to a preferred embodiment, the support is silica.

According to another embodiment of the process of the present invention, the support is at least an anionic laminar compound of hydrotalcite type or its derivatives.

According to another embodiment of the process of the present invention, the support is active carbon.

According to another embodiment of the process of the present invention, the support is at least an organic polymer. This support may be a copolymer selected from among a styrene copolymer, 4-hydroxyethylstyrene copolymer and 4-glycidylstyrene copolymer. Also, according to another embodiment, the support is a polymer of dendrimer type which may be selected between PAMAM and PEI types.

According to a particular embodiment of the present invention, the catalyst may further comprise at least one metal of groups 9, 10 and 11 of the periodic table, preferably gold.

The size of the gold nanoparticles is of vital importance in the catalytic activity, since the activity of the catalyst decreases strongly when the size of the gold particles is greater than 20 nm. The size of the suitable particle for observing the catalysis of the oxidizing reaction of amines with oxygen should be, preferably, between 1 nm and 20 nm, and particularly preferably, between 2 nm and 10 nm. In addition, the gold is approximately in a percentage between 0.01% and 10%, preferably between 0.05% and 6% by weight with respect to the catalyst.

As we have already seen, the metal oxide in a preferred embodiment of the present invention may contain gold nanoparticles. When the gold particles are supported, the nature of the support affects the final activity and selectivity of the catalyst. Those gold nanoparticles are preferably supported in an insoluble material which may be an organic polymer or active carbon or even an organic-inorganic support such as, for example a metal-organic framework (MOF) material. Surprisingly, the support does not show too great activity (as is the case of some organic polymers, active carbon and MOF), so the catalytic activity in these cases derives almost completely from the gold nanoparticles. In the cases of gold nanoparticles supported in metal oxides, the catalytic activity of the metal oxide is notably increased by the gold nanoparticles. These metal oxides may also by themselves have certain catalytic activity in the oxidation reaction of aromatic amines with oxygen in order to form azobenzenes. However, as we have already stated, the activity measured as the reaction rate increases significantly with respect to the azo compound when the nanoparticulated oxide is modified by the adsorption of gold nanoparticles on its surface. The zeolites and the structured mesoporous oxides with or without subsequent treatments, or the combinations between them, may also be suitable supports.

For example, in the absence of gold nanoparticles, certain oxides, such as cerium oxide, titanium oxide and iron oxide, give lower conversions for the same period of time than their analogs containing gold nanoparticles. Thus, when gold is added to the support, which forms part of the catalyst, in amounts between 0.01% and 10%, and most preferably between 0.05% and 6% by weight with respect to the catalyst, the activity and selectivity of the catalyst become surprisingly greater. The behaviour of the catalyst, in particular with metal oxides, in the described reactions can be neither deduced nor derived from the knowledge of the state of the art in catalysis using nanoparticles of metal oxides containing or not containing gold. (ASK Hashmi, J Hutchings Graham: Gold catalysis. Angew. Chem. Int. Ed. 45 (2006) 7896-936; SAK Hashmi: Gold-catalyzed organic reactions. Chemical Reviews 107 (2007) 3180-211; T Mallat, A Baiker: Chem. Rev. 104 (2004) 3037). In the present invention, organic polymers interacting with the gold nanoparticles by means of forces of the metal -atom type, which capture and immobilize the gold nanoparticles by interlinking the polymeric chains around the nanoparticles, are claimed, among others, as supports for the gold nanoparticles. For example, polystyrene or a copolymer, which comprises styrene and derivatives as monomers, can be used as a support. A preferred catalyst is the one in which three co-monomers (styrene, p-glycidylstyrene and p-2-hydroxyethylstyrene) form a polymeric support. The proportions of the monomers can vary within a wide range, with the preferred proportion of styrene/ p-glycidylstyrene/p-2-hydroxyethylstyrene being 90/7/3.

Polystyrenes containing other co-monomers, neutral or positively or negatively charged, polyacrylates and polyacrylamides are other polymers that can be used as supports. Soluble polymers of dendrimer type can also serve as supports of the gold nanoparticles. The preferred supports among dendrimers are those that contain nitrogen atoms, such as the polyethylenimine and the polyamidoamine of third and fourth generation.

Another type of support is the active carbons of different origin and chemical composition. These supports improve their effectiveness by enlarging the area. Other insoluble supports where the gold nanoparticles can be deposited are also hybrid organic-inorganic materials, and in particular metal-organic framework (MOF) materials. In the latter crystalline, micro-/mesoporous materials formed by interactions between a metal or sub-nanometric metal aggregates and organic linkers, the gold nanoparticles can occupy exterior or interior positions with respect to the MOF crystal.

Also, as it has already been mentioned above, other gold nanoparticle supports are metal oxides, both amorphous and crystalline and structured, both laminar and with porosity in the range of 0.7 nm to 100 nm. These oxides, which are used as supports, may contain two or more metals. The crystallographic phase in any of the metal oxides may be pure or a mixture in any proportion. The particle size of the support can be from several nanometres to several microns. The oxide can be stechiometric or the proportion between the metal and the oxygen may differ from that expected depending on the valences of the elements. Thus a preferred embodiment of the present invention consists of 0.1%-4% by weight of gold with crystal size between 2 nm and 10 nm on titanium oxide with average particle size of 25 nanometres and in its anatase phase.

In addition, as it has already been pointed out, the metal oxides with or without gold nanoparticles may contain, in small proportions, other metals and non-metal elements acting as doping agents. In a preferred embodiment of the present invention, titanium oxide, predominantly in anatase phase, is doped with 0.5% iron, and the resulting solid (Fe(0.5)/TiO₂) shows improved activity for azobenzene formation with respect to titanium oxide without doping. In the same way, it is possible to dope the TiO₂ with copper at a percentage of less than 1% by weight.

The supports can also be amorphous, but they can have also a type of structure. For example, the supports may be laminar such as materials of hydrotalcite type (oxides mixed with a divalent metal and another trivalent one), or may be derived from mixed oxides.

As mentioned above, the process of the present invention comprises an amine or polyamine as a starting compound for obtaining the corresponding azo compound. The amines that may be used in the framework of the present invention are aliphatic or aromatic amines and polyamines. Particularly suitable are those amines which have substituents that increase the electronic density in the nitrogen. Thus, in the case of aromatic amines, those possessing donor groups of electrons attached to the aromatic nucleus, are the ones that react with higher speed.
Generally, the amine or polyamine may be an amine with the formula R(NH₂)ₙ
where R is selected from among the group consisting of a substituted or non-substituted alkyl with 1 to 20 carbon atoms, substituted or non-substituted aryl with 6 to 15 carbon atoms, substituted or non-substituted arylalkyl with 7 to 15 carbon atoms, substituted or non-substituted alkenyl with 2 to 20 carbon atoms, substituted or non-substituted alkinyl with 2 to 20 carbon atoms, substituted or non-substituted cycloalkyl with 3 to 20 carbon atoms, substituted or non-substituted cycloalkenyl with 4 to 20 carbon atoms and substituted or non-substituted cycloalkinyl with 5 to 20 carbon atoms; and n is 1, 2, 3, 4, 5 or 6. Preferably, the amine or polyamine is selected from among n-propylamine, isopropylamine, α-cyano isopropylamine, n-butylamine, n-hexylamine, n-octylamine, laurylamine, cyclopentylamine, cyclohexylamine, cyclooctylamine, 1,2-diaminoethane, 1,12-diaminododecane, 1,4-diaminocyclohexane, 1,3-bis(aminomethyl)cyclohexane, 1,4-bis(aminomethyl)cyclohexane, hydrogenated 2,4-diaminodiphenylmethane, hydrogenated toluilenediamine, aniline, benzylamine, α-cyanobenzylamine, α-cyano-α-phenylethanamine, 2-aminotoluene, 4-aminotoluene, 2,4'-diaminodiphenylmethane, 4,4'-diaminodiphenylmethane, 2,2'-diaminodiphenylmethane, 2,4-toluenediamine, 2,6-toluenediamine, p-phenylendiamine, m-phenylendiamine, 4- (N,N-dimethylamino)aniline, 4-hydroxyaniline, 4-methoxyaniline, α-aminonaphthalene, β-aminonaphthalene, 9-aminoanthracene, 1,5-diaminonaphthalene and mixtures thereof.

The mixture of two amines in suitable proportions makes it possible to form asymmetric azo compounds where groups R¹ and R² of Figure 1 are different. Particularly important is the case when the two groups R¹ and R² are different aromatic groups. These asymmetric azo compounds can be obtained with high selectivity when a mixture of amines is treated with oxygen in the presence of catalysts of the present invention (see examples 6 and 7). The selectivity with respect to the formation of an asymmetric azo compound is particularly high when one of the amines is rich in electrons and the other one is deficient. In aromatic amines derived from benzene, this difference in the electronic density of the nitrogen atoms may be determined on the basis of the electron-donor or electron-acceptor nature of the substituents present in the aromatic nucleus.

According to the process of the present invention, it is also possible to design continuous processes by using, for example, a fixed bed reactor using a supported gold catalyst. The azo compounds of the present invention may be transformed into other compounds with a higher or lower oxidation level such as, for example, between other azoxy and nitro compounds and hydrazines and amines.

According to a preferred embodiment, the amine source may be aniline that is oxidized to azobenzene.

According to another preferred embodiment, the amine source may be (4,4-dimethylamino)aniline, and it is oxidized in order to obtain *butter yellow.*

According to another preferred embodiment, the starting product is a mixture of (4,4-dimethylamine)aniline and sodium 4-aminobenzenesulfonate and *methyl orange* is obtained.

According to another preferred embodiment, it starts from cyanoisopropylamine which is oxidized in order to obtain azo bis(isobutyronitrile).

### EXAMPLES

Non-limiting examples of the present invention are described below.

### Example 1

***Formation of azobenzene by oxidation with oxygen of a solution of aniline in toluene using nanoparticulated titanium oxide.*** In a reinforced glass reactor equipped with a pressure and temperature controller, 93.13 mg of aniline are dissolved in 2 ml of toluene in the presence of 129 mg of TiO₂. The reactor is purged 3 times with 5 bar of oxygen and is then filled with the desired pressure of O₂ (5 bar) completely submerging it in a paraffin bath preheated to 100 °-C, and the suspension is magnetically stirred. During the experiment, the O₂ pressure is maintained constant at 5 bar, supplying more oxygen when appropriate. The azobenzene formation at different times of the reaction can be established by analyzing an aliquot from the reactor. These aliquots are centrifuged to eliminate the catalyst particles, and the residue is analyzed with GC-MS. The yield of azobenzene is 52.5% after 24 hours.

### Example 2

### Formation of azobenzene by oxidation of aniline with oxygen in the absence of a solvent using nanoparticulated titanium oxide.

A total of 2 g of aniline and 129 mg of TiO₂ are put in a reinforced glass reactor equipped with a pressure and temperature controller. The reactor is purged 3 times with 5 bar of oxygen and it is then hermetically closed by loading it with a pressure of O₂ of 5 bar. The reactor is completely submerged in a paraffin bath preheated to 100 ºC and is magnetically stirred. During the experiment, the O₂ pressure is maintained at 5 bar by supplying more oxygen when the pressure falls to 3 bars. The temporal evolution of the products can be determined by analyzing aliquots from the reactor at different times. These aliquots are dissolved in acetonitrile and are centrifuged to eliminate the catalyst particles, and the residue is analyzed with GC-MS. After 72 hours, the yield of azobenzene is 58%.

### Example 3

### Formation of azobenzene by oxidation of aniline with oxygen in the absence of a solvent using gold nanoparticles added to a polymeric matrix as a catalyst.

The catalyst used in this example relates to a copolymer of three monomers, styrene/p-glycidylstyrene/p-2-hidroxyethylstyrene in a proportion of 90/7/3 containing gold nanoparticles (1.5% by weight, average size of 5 nm). This catalyst is prepared by forming the polymer by means of radicals, synthesizing the gold nanoparticles by reduction of AuCl₄⁻ and subsequent interlinking of epoxy and alcohol groups according to the process described in the literature and known in the state of the art (Miyamura, H.; Matsubara, R.; Miyazaki, Y.; Kobayashi, S. Angew. Chem. Int. Ed. 2007, 46, 4151). A total of 2 g of aniline and the amount of gold catalyst supported in a polymeric matrix for achieving a molar gold/aniline reaction of 1 mol% are put in a reinforced glass reactor equipped with a pressure and temperature controller. The reactor is purged 3 times with 5 bar of oxygen and it is then closed hermetically and is pressurized with oxygen with a final pressure of 5 bar. The reactor is completely submerged in a paraffin bath preheated to 100 ºC and is magnetically stirred. During the experiment, the O₂ pressure is maintained around 5 bar by supplying more oxygen when appropriate. The progress of the reaction and the temporal evolution are determined by analyzing the aliquots at different times. The samples from the reactor are dissolved in acetonitrile and are centrifuged to eliminate the catalyst particles, and the residue is analyzed with GC-MS. After 6 days, the conversion is 50%.

### Example 4

### Formation of azobenzene from nitrobenzene by consecutively completing its hydrogenation to aniline and aerobic oxidation by using gold nanoparticles supported on nanoparticulated titanium oxide.

The process is performed by using a single catalyst in two stages with the first one consisting of transformation of the nitrobenzene into aniline and the second one into oxidation of the aniline to azobenzene.

As a first stage, 123 mg of nitrobenzene are dissolved in 2 ml of toluene in a reinforced glass reactor equipped with a pressure and temperature controller, adding 131 mg of titanium oxide catalyst (Degusa P25) containing 1.5% by weight of gold nanoparticles (average size of 5 nm). This catalyst is prepared by means of precipitation-deposition on P25 starting from aqueous solutions of HAuCl₄ where the pH is increased by a factor greater than 5 (typically 7) according to the process described in the literature (Abad, A.; Corma, A.; Garcia, H. Chem. Eur. J. 2008, 14, 212.) The reactor containing the nitrobenzene and the catalyst is purged 3 times with 5 bar of hydrogen and is then loaded with a pressure of 9 bar of H₂. The reactor is completely submerged in a paraffin bath preheated to 120 ºC and is magnetically stirred. During the experiment, the H₂ pressure is maintained at a value greater than 6 bar by supplying more hydrogen when appropriate. The progress of the reaction is followed by analyzing aliquots from the reactor at different times. The samples are centrifuged to eliminate the catalyst particles, and the residue is analyzed with GC-MS. After 6 hours, the yield of the aniline is 94.5%.

In the second stage, in the same reactor, the temperature of the bath is reduced to 100 ºC and all hydrogen from the reactor is eliminated by purging the system 3 times with 5 bar of oxygen. Finally, the reactor is loaded with molecular oxygen with a pressure of 5 bar and, in the same way as in the previous stage, the O₂ pressure is maintained at a value greater than 3 bar, adding more oxygen when appropriate. The progress of the reaction is determined by analyzing aliquots at different times. The samples are centrifuged to eliminate the catalyst particles, and the residue is analyzed with GC-MS. After 9 hours, the yield of azobenzene is 92%.

### Example 5

### Preparation of the butter yellow by means of aerobic oxidation of N,N-dimethylaminoaniline in the presence of gold nanoparticles supported on titanium oxide.

A total of 136 mg of 4-N,N-dimethylaminoanilin are dissolved in 2 ml of toluene in a reinforced glass reactor equipped with a pressure and temperature controller, and 131 mg of a catalyst consisting of nanoparticulated titanium oxide (average particle size of 25 nm, 80% anatase and 20% rutile) and containing 1.5% by weight of gold nanoparticles (average size of 5 nm) are added to the solution. This catalyst is the same one used in example 4. The reactor is purged 3 times with 5 bar of oxygen and is then loaded with a final oxygen pressure of 5 bar. The reactor is completely submerged in a paraffin bath preheated to 100 ºC and is magnetically stirred. During the experiment, the O₂ pressure is maintained at a value greater than 3 bar by supplying more oxygen when appropriate. The progress of the reaction is determined by analyzing aliquots of the crude of the reaction at different times. The samples are centrifuged to eliminate the catalyst particles, and the residue is analyzed with GC-MS. After 16 hours, 99% yield of butter yellow [(4,4'-bis(N,N-dimethylamino)azobenzene] is obtained.

### Example 6

### Preparation of methyl orange by aerobic oxidation of a mixture of N,N-dimethylaminoaniline and sodium 4-aminobenzenesulfonate in the presence of gold nanoparticles supported on titanium oxide.

A total of 68 mg of 4-N,N-dimethylaminoaniline and 97.5 mg of sodium 4-aminobenzenesulfonate are dissolved in 2 ml of toluene in a reinforced glass reactor equipped with a pressure and temperature controller; 131 mg of a catalyst consisting of titanium nanoparticles (Degusa, P25) and containing gold nanoparticles are added to the solution. This catalyst is the same as the one used in examples 4 and 5. The system is purged 3 times with 5 bar of oxygen and is finally loaded with an O₂ pressure of 5 bar. The reactor is completely submerged in a paraffin bath preheated to 100 ºC and is magnetically stirred. During the experiment, the O₂ pressure is maintained at a value greater than 3 bar, supplying more oxygen when appropriate. After 40 hours, the reaction is stopped, the crude is centrifuged in order to eliminate the catalyst, and the residue is dried. The solid residue is re-crystallized in a small volume of hot toluene. The resulting crystals are filtered, rinsed with cold ether and are dried throughout the night at reduced pressure. A 92% yield of methyl orange is obtained which must show its purity based on its NMR ¹H, NMR ¹³C, UV electroscopic properties and elementary analysis.

### Example 7

### Preparation of 2-(phenylazo)pyridine by aerobic oxidation of a mixture of aniline and 2-aminopyridine in the presence of gold nanoparticles supported on titanium oxide.

A total of 46.5 mg of aniline and 47 mg of 2-aminopyridine are dissolved in 2 ml of toluene in a reinforced glass reactor equipped with a pressure and temperature controller; 131 mg of a catalyst consisting of titanium nanoparticles (Degusa, P25) containing 1.5% by weight gold nanoparticles (average size of 5 nm) are added to this solution. This catalyst is prepared by using the precipitation/deposition process that is well known in the state of the art, and it is the same catalyst described in examples 4, 5 and 6. The system is purged 3 times with 5 bar of oxygen and is finally loaded with an O₂ pressure of 5 bar. The reactor is completely submerged in a paraffin bath preheated to 100 ºC and is magnetically stirred. During the experiment, the O₂ pressure is maintained at a value greater than 3 bar by supplying more oxygen when appropriate. The progress of the reaction is determined by analyzing aliquots at different times. The samples are centrifuged to eliminate the catalyst particles, and the residue is analyzed with GC-MS. After 40 hours, 52% yield of the 2-(phenylazo)pyridine is obtained.

### Example 8

### Preparation of azo bis(isobutyronitrile) by means of oxidation of 1-cyano-1-methylethanamine in the presence of gold nanoparticles supported on titanium oxide.

A total of 84.12 mg of 1-cyano-1-methylethanamine are dissolved in 2 ml of toluene in a reinforced glass reactor equipped with a pressure and temperature controller; 131 mg of the gold catalyst supported in titanium oxide, which has been used in examples 4-7, are added to this solution. The system is purged 3 times with 5 bar of oxygen and is finally loaded with oxygen at a pressure of 5 bar. The reactor is completely submerged in a paraffin bath preheated to 100 ºC and is magnetically stirred. During the experiment, the O₂ pressure is maintained at a value greater than 3 bar, supplying more oxygen when appropriate. The progress of the reaction is determined by analyzing aliquots at different times. The samples are centrifuged to eliminate the catalyst particles, and the product is analyzed with GC-MS. After 40 hours, 46% yield of the azo bis(isobutyronitrile) is obtained.

## Claims

1. Preparation process of azo compounds, **characterized in that** the reaction comprises at least:
- one amine or polyamine,
- molecular oxygen,
- a catalyst formed by at least one support selected from among at least one metal oxide of one of the elements of groups 3, 4, 5, 6, 8, 9, 11 and 13, silica, anionic laminar compound of the hydrotalcite type or its derivatives, active carbon or an organic polymer.

2. Preparation process of azo compounds according to Claim 1, **characterized in that** the catalyst support is a metal oxide of one of the elements of groups 3, 4, 5, 6, 8, 9, 11 and 13.

3. Process according to Claim 2, **characterized in that** the metal oxide is selected from among at least one of the following oxides: TiO₂, CeₓO_{y}, Al₂O₃, MgO, CaO, CuₓO_{y}, CoₓO_{y}, FeₓO_{y}, CrₓO_{y}, Y₂O₃, ZrO₂ and combinations thereof.

4. Process according to Claim 3, **characterized in that** the metal oxide is titanium oxide.

5. Process according to Claim 3, **characterized in that** the metal oxide is cerium oxide.

6. Process according to Claim 3, **characterized in that** the metal oxide is yttrium oxide.

7. Process according to Claim 3, **characterized in that** the metal oxide is iron oxide.

8. Process according to Claim 3, **characterized in that** the metal oxide is zirconium oxide.

9. Process according to any of the preceding claims **characterized in that** the metal oxide further contains at least one metal or non-metal element, which acts as a doping agent, selected from among groups 9, 10, 11, and 12 and combinations thereof.

10. Process according to Claim 1, **characterized in that** the support is silica.

11. Process according to Claim 1, **characterized in that** the support is at least an anionic laminar compound of hydrotalcite type or its derivatives.

12. Process according to Claim 1, **characterized in that** the support is active carbon.

13. Process according to Claim 1, **characterized in that** the support is at least an organic polymer.

14. Process according to Claim 13, **characterized in that** the support is a copolymer selected from among a copolymer of styrene, 4-hydroxyethylstyrene copolymer and 4-glycidylstyrene copolymer.

15. Process according to Claim 13, **characterized in that** the support is a polymer of dendrimer type.

16. Process according to Claim 15, **characterized in that** the support is of PAMAM type.

17. Process according to Claim 15, **characterized in that** the support is of PEI type.

18. Process according to any of the preceding claims, **characterized in that** the catalyst further comprises at least one metal of groups 9, 10 and 11 of the periodic system.

19. Process according to Claim 18, **characterized in that** the metal is gold.

20. Process according to Claim 19, **characterized in that** the particle size of the gold is between 1 nm and 20 nm.

21. Process according to any of claims 19 and 20, **characterized in that** the percentage of the gold is between 0.01% and 10% by weight with respect to the catalyst.

22. Process according to Claim 21, **characterized in that** the percentage of the gold is between 0.05% and 6% by weight with respect to the catalyst.

23. Process according to any of the preceding claims, **characterized in that** the amine or polyamine is an amine with the formula
R(NH₂)n
where R is selected from among the group consisting of substituted or non-substituted alkyl with 1 to 20 carbon atoms, substituted or non-substituted aryl with 6 to 15 carbon atoms, substituted or non-substituted arylalkyl with 7 to 15 carbon atoms, substituted or non-substituted alkenyl with 2 to 20 carbon atoms, substituted or non-substituted alkinyl with 2 to 20 carbon atoms, substituted or non-substituted cycloalkyl with 3 to 20 carbon atoms, substituted or non-substituted cycloalkenyl with 4 to 20 carbon atoms and substituted or non-substituted cycloalkinyl with 5 to 20 carbon atoms; and n is 1, 2, 3, 4, 5 or 6.

24. Process according to Claim 23, **characterized in that** the amine or polyamine is selected from among n-propylamine, isopropylamine, α-cyano isopropylamine, n-butylamine, n-hexylamine, n-octylamine, laurylamine, cyclopentylamine, cyclohexylamine, cyclooctylamine, 1,2-diaminoethane, 1,12-diaminododecane, 1,4-diaminocyclohexane, 1,3-bis(aminomethyl)cyclohexane, 1,4-bis(aminomethyl)cyclohexane, hydrogenated 2,4-diaminodiphenylmethane, hydrogenated toluilenediamine, aniline, benzylamine, α-cyanobenzylamine, α-cyano-α-phenylethanamine, 2-aminotoluene, 4-aminotoluene, 2,4'-diaminodiphenylmethane, 4,4'-diaminodiphenylmethane, 2,2'-diaminodiphenylmethane, 2,4-toluenediamine, 2,6-toluenediamine, p-phenylendiamine, m-phenylendiamine, 4- (N,N-dimethylamino)aniline, 4-hydroxyaniline, 4-methoxyaniline, α-aminonaphthalene, β-aminonaphthalene, 9-aminoanthracene, 1,5-diaminonaphthalene and mixtures thereof.

25. Process according to any of the preceding claims, **characterized in that** the amine is aniline and is oxidized to azobenzene.

26. Process according to any of the preceding claims, **characterized in that** the amine is (4,4-dimethylamino)aniline and is oxidized to *butter yellow* or *methyl orange.*

27. Process according to any of the preceding claims, **characterized in that** the amine is □̃ cyanoisopropylamine and is oxidized to azo bis(isobutyronitrile).
